# EUROPEAN PATENT SPECIFICATION

(11) **EP 1 901 055 B1**
(45) Date of publication and mention of the grant of the patent: **18.10.2017**
(21) Application number: 06019385.1
(22) Date of filing: 15.09.2006
(51) Int. Cl.: G01N 19/02, A61F 2/00

(54) **Method of measuring the retention force of a biomedical specimen**
Verfahren zur Haltekraftmessung eines Implantats
Procédé pour mesurer une force de rétention d'un implant

(43) Date of publication of application: 19.03.2008
(73) Proprietor: Abbott Laboratories Vascular Enterprises Limited, Dublin 2 (IE)
(72) Inventor: Zscheeg, Harry Dr., 78239 Rielasingen-Worblingen (DE)
(74) Representative: Hoefer & Partner Patentanwälte mbB

(56) References cited:
- DE-A1- 19 803 219
- JP-A- 2004 177 332
- BASHAR ET AL: "Mechanical Properties of Various Z-Stent Designs: An Endovascular Stent-Grafting Perspective" ARTIFICIAL ORGANS, vol. 27, no. 8, August 2003 (2003-08), pages 714-721, XP002421653
- ROGERS C RITTER ET AL: "Measurement of Friction on Straight Catheters in in vitro Brain and Phantom Material" IEEE TRANSACTIONS ON BIOMEDICAL ENGINEERING, IEEE SERVICE CENTER, PISCATAWAY, NJ, US, vol. 45, no. 4, April 1998 (1998-04), XP011006527 ISSN: 0018-9294
- FRANK G. SHELLOCK: "Biomedical Implants and Devices: Assessement of Magnetic Field Interactions With a 3.0-Tesla MR System" JOURNAL OF MAGNETIC RESONANCE IMAGING, vol. 16, December 2002 (2002-12), pages 721-732, XP002421654

## Description

The present invention concerns a method of measuring a retention force of a biomedical specimen in the form of an electrically conductive structure located on a carrier according to claim 1.

According to the present invention, such electrically conductive structures can be stents, markers, sleeves with or without braidings as used e.g in delivery systems to deliver a self-expandable stent, and similar biomedical components.

Formerly known measuring methods are all based on mechanical principles that, however, encounter the disadvantage of a partly heavy impact on the structure to be measured and thus on the values measured.

It is, therefore, an objective of the present invention to provide a measuring method that makes it possible to determine the retention force of a biomedical specimen located on a carrier without affecting the specimen itself and thus the measurement negatively.

The solution of this objective is achieved by the features of claim 1. The dependent claims contain advantageous embodiments of the present invention.

In order to avoid the aforementioned disadvantages of prior art systems that include e.g. an unwanted axial compression of the specimen or a test-originating added force on the system, the present invention principally proposes the use of external force fields to assert a force on the specimen. Electric currents are induced in the specimen to make it behave like an electromagnet. An external magnetic field is used to assert a force on the specimen such that it is moved off the carrier structure, e.g. in an axial direction. The test setup parameters (like current, voltage etc.) can be used to calculate the applied force, and thus the retention force can be determined.

According to the present invention, the measuring or determination of the retention force can be achieved by the detection of a movement onset of the specimen. This means the force that is exerted on the specimen overcompensates the retention force.

Also, the principles of the present invention include the measuring of parameters that cause a specimen movement and are controlled such that the specimen velocity stays constant (e.g. the parameter current that causes the magnetic field which, in turn, causes the specimen movement) on the carrier structure what corresponds to a measurement of the dynamic friction force.

An advantage of the method according to the present invention is that only the following modules are necessary:
- a generating device for a varying magnetic field(bearing in time, space or both), e.g. an electromagnet,
- a specimen movement detection system, and
- optionally, but not mandatory, an electronic feedback loop that could be utilized to generate a constant movement velocity of the specimen by controlling the operating parameters of the magnetic field (dynamic friction force measurement).

The feedback loop could e.g. be based on an optical movement detection. Moreover, because the current that is induced in the specimen is dependent on the specimen's impedance, an impedance measurement can be performed on the specimen. When comparing specimens of different impedances, the different values must be considered when interpreting the test results.

The following possible, but not exclusive, realizations of the present invention can be used:

### Test Setup 1

An electromagnet is used to generate an alternating magnetic field (magnetic field changes in time). The specimen might be disposed near or inside the electromagnet. The magnetic field's amplitude or frequency or both are increased until the specimen starts to move. The magnetic force and thus the operational parameters of the electromagnet needed to start the specimen movement are correlated with the magnitude of friction holding the specimen in place (retention force).

### Test Setup 2

The specimen can represent a secondary coil of a transformer. The primary coil e.g. could be located on a straight carrier used as a core.

According to this embodiment, the specimen would be located on the core also.

E.g. the core could be shaped such that an instrument with the specimen on it could be shoved over the core. Changing the magnetic field in the primary coil, e.g. by switching on the supply voltage, induces a voltage in the specimen and thus a force (magnetic field changes in time).

The supply voltage can be switched on and off with stepwise increased amplitude until the specimen moves. The voltage amplitude applied serves as a measure of the retention force of the specimen on the carrier structure.

### Test Setup 3

The specimen on the carrier structure is moved into a magnetic field that has a spatial gradient. For moving the specimen e.g. a linear drive or another kind of drive can be used.

Instead of moving the specimen, the magnetic field generating equipment can also be moved.

The force exerted on the specimen can be increased by increasing the relative movement velocity until the retention force is reached and the specimen begins to move relative to the carrier structure.

Because it is not necessary that the magnetic field varies in time, but spatially only, permanent magnets can be utilized with this embodiment instead of electromagnets.

### Test Setup 4

Equivalent to test setup 3 including the following changes:
The magnetic field does not need to have a gradient. Instead, the movement of the specimen into the field is of the accelerated type. Thus, also an increasing force on the specimen is generated.

Moreover, it is possible to use several external magnets to gain a better control of the forces applied.

Obviously, the method according to the present invention works for any specimen comprising any kind of material that conducts electric current. Exemplary materials that conduct electric current comprise metals and metal alloys like stainless steel, CoCr alloys, NiTi alloys, NbTa alloys, NbWn alloys, TaWn alloys, Pt, Ir, Au and alloys thereof as well as dual-layers, tri-layers or multi-layers or blends of any of these materials. The specimen can be made from electric current conducting material or can comprise electric current conducting material, i.e. the specimen can be made in part out of electric current conducting material. The specimen can for example comprise a layer, a carrier used as a core or a braiding made out of electric current conducting material.

Further features, advantages and principles of the present invention will become apparent from the following description of the drawings, in which:
- Fig. 1: is a schematically simplified depiction of a first embodiment of the principle of the present invention,
- Fig. 2: is a simplified depiction of a second embodiment, and
- Fig. 3: is a simplified depiction of a third embodiment of the method according to the present invention.

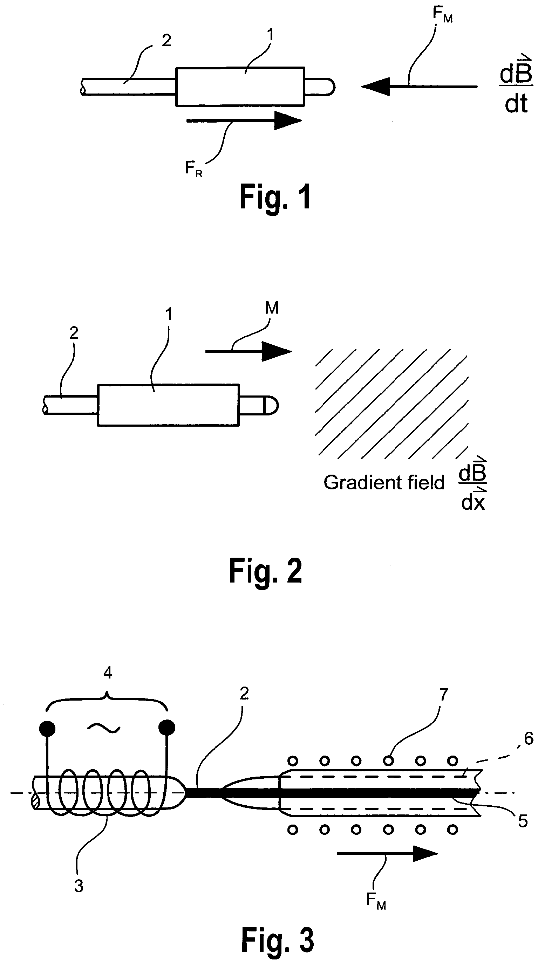

Fig. 1 shows in a strongly simplified way a biomedical specimen in the form of an electrically conductive structure such as a stent 1 that is located on a carrier 2 that may e.g. be a balloon catheter. An external alternating magnetic field dB/dt is created near or around the specimen 1 that creates a magnetic force F_{M} counteracting a retention force F_{R} that is e.g. a frictional force that holds the specimen 1 on the carrier 2.

In general, Fig. 2 shows a similar embodiment of a specimen 1 on a carrier 2 that, however, is moved in the direction of the arrow M into a magnetic field dB/dt that is a magnetic field having a spatial gradient.

Fig. 3 depicts a third possible embodiment of the present invention that corresponds to test setup 2 explained hereinbefore.

Accordingly, this embodiment comprises again a carrier 2 that is surrounded by a primary coil 3 being connected to a voltage supply 4.

According to this embodiment, an arrangement of a shaft 5, a catheter 6 as well as a stent 7 is disposed on carrier 2 so that this arrangement is comparable to a transformer having a primary coil 3 and stent 7 as a secondary coil.

So, changing the magnetic field in the primary coil 3 induces a voltage in the specimen (in this case stent 7) and thus a force F_{M}.

### List of Reference Signs

- 1: Specimen (conductive structure in the form of a stent, marker, sleeve etc.)
- 2: carrier or carrier used as a core
- 3: Coil
- 4: Voltage supply
- 5: Shaft
- 6: Catheter
- 7: Secondary coil (stent or the like)
- F_{M}: Magnetic force
- F_{R}: Retention force
- dB/dt: Magnetic field changing timewise
- dB/dx: Magnetic field with a spatial gradient

## Claims

1. Method of measuring the retention force (F_{R}) of a biomedical specimen (1) in the form of an electrically conductive structure located on a carrier (2), comprising the following method steps:
- creating an external alternating magnetic field dB/dt or dB/dx near the specimen (1);
- increasing the amplitude and/or frequency of the magnetic field;
- detecting the start of movement of the specimen (1) on the carrier (2); and
- calculating the retention force (F_{R}) by determining the applied magnetic force (F_{M}) on the basis of the parameters of the respective magnetic field.

2. Method according to claim 1, comprising the following steps:
- locating a primary coil (3) on the carrier (2);
- locating the specimen (7) on the same carrier (2) thus creating a transformer-like arrangement;
- creating the external alternating magnetic field in the primary coil (3) thus applying a magnetic force (F_{M}) on the specimen (7) by magnetic induction.

3. Method according to claim 1, being **characterized by** moving the specimen (1; 7) and the carrier (2) relative to the magnetic field dB/dx having a spatial gradient.

4. Method according to claim 1, **characterized by** using permanent magnets to create the magnetic field dB/dx varying spatially.

## Patentansprüche

1. Verfahren zum Messen der Haltekraft (F_{R}) einer biomedizinischen Probe (1) in der Form einer elektrisch leitfähigen Anordnung, die auf einem Träger (2) angeordnet ist, das die folgenden Verfahrensschritte aufweist:
- Erzeugen eines äußeren magnetischen Wechselfeldes dB/dt oder dB/dx in der Nähe der Probe (1);
- Erhöhen der Amplitude und/oder Frequenz des Magnetfeldes;
- Erfassen des Beginns einer Bewegung der Probe (1) auf dem Träger (2); und
- Berechnen der Haltekraft (F_{R}) durch Bestimmen der aufgebrachten Magnetkraft (F_{M}) auf der Basis der Parameter des jeweiligen Magnetfeldes.

2. Verfahren gemäß Anspruch 1, das die folgenden Schritte aufweist:
- Anordnen einer Primärspule (3) auf dem Träger (2);
- Anordnen der Probe (7) auf demselben Träger (2), wodurch eine transformatorähnliche Anordnung erzeugt wird;
- Erzeugen des äußeren magnetischen Wechselfeldes in der Primärspule (3), wodurch eine Magnetkraft (F_{M}) auf die Probe (7) durch magnetische Induktion aufgebracht wird.

3. Verfahren gemäß Anspruch 1, das durch Bewegen der Probe (1;7) und des Trägers (2) relativ zum Magnetfeld (dB/dx), das einen räumlichen Gradienten aufweist, gekennzeichnet ist.

4. Verfahren gemäß Anspruch 1, **gekennzeichnet durch** Verwendung von Permanentmagneten, um das räumlich variierende Magnetfeld (dB/dx) zu erzeugen.

## Revendications

1. Procédé de mesure de la force de rétention (F_{R}) d'un échantillon biomédical (1) sous la forme d'une structure électroconductrice située sur un support (2), comprenant les étapes de procédé suivantes :
- la création d'un champ magnétique alternatif externe dB/dt ou dB/dx à proximité de l'échantillon (1) ;
- l'augmentation de l'amplitude et/ou de la fréquence du champ magnétique ;
- la détection du début du déplacement de l'échantillon (1) sur le support (2) ; et
- le calcul de la force de rétention (F_{R}) par la détermination de la force magnétique appliquée (F_{M}) sur la base des paramètres du champ magnétique respectif.

2. Procédé selon la revendication 1, comprenant les étapes suivantes :
- le placement d'une bobine principale (3) sur le support (2) ;
- le placement de l'échantillon (7) sur le même support (2) pour ainsi créer un agencement de type transformateur ;
- la création du champ magnétique alternatif externe dans la bobine principale (3) pour ainsi appliquer une force magnétique (F_{M}) sur l'échantillon (7) par induction magnétique.

3. Procédé selon la revendication 1, **caractérisé par** le déplacement de l'échantillon (1 ; 7) et du support (2) par rapport au champ magnétique dB/dx présentant un gradient spatial.

4. Procédé selon la revendication 1, **caractérisé par** l'utilisation d'aimants permanents pour créer le champ magnétique dB/dx variant dans l'espace.
